# EUROPEAN PATENT SPECIFICATION

(11) **EP 1 159 274 B1**
(45) Date of publication and mention of the grant of the patent: **26.03.2003**
(21) Application number: 99968622.3
(22) Date of filing: 22.11.1999
(51) Int. Cl.: C07D 307/87

(54) **METHOD FOR THE PREPARATION OF CITALOPRAM**
VERFAHREN ZUR HERSTELLUNG VON CITALOPRAM
PROCEDE DE PREPARATION DE CITALOPRAME

(30) Priority: 25.06.1999 DK 99920
(43) Date of publication of application: 05.12.2001
(73) Proprietor: H. Lundbeck A/S, 2500 Valby-Copenhagen (DK)
(72) Inventor: PETERSEN, Hans, DK-2720 Vanlose (DK); ROCK, Michael, Harold, DK-2650 Hvidovre (DK); SVANE, Henrik, DK-2830 Virum (DK)
(74) Representative: Meidahl Petersen, John
(86) International application number: DK9900640
(87) International publication number: WO00013648

(56) References cited:
- US-A- 4 136 193

## Description

The present invention relates to a method for the preparation of the well known antidepressant drug citalopram,1-[3-(dimethylamino)propyl]-1-(4-fluorophenyl)-1,3-dihydro-5-isobenzofurancarbonitrile.

### Background of the Invention.

Citalopram is a well known antidepressant drug that has now been on the market for some years and has the following structure:

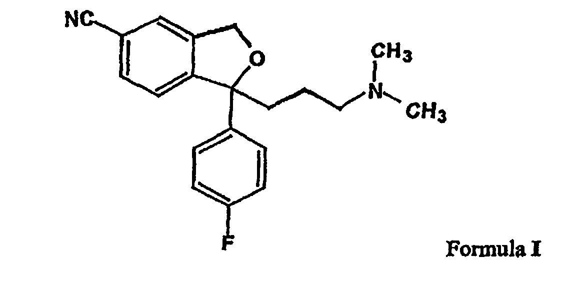

It is a selective, centrally acting serotonin (5-hydroxytryptamine; 5-HT) reuptake inhibitor, accordingly having antidepressant activities. The antidepressant activity of the compound has been reported in several publications, eg. J. Hyttel, *Prog. Neuro-Psychopharmacol. & Biol. Psychiat*., **1982**, *6*, 277-295 and A. Gravem, *Acta Psychiatr. Scand*., **1987**, *75*, 478-486. The compound has further been disclosed to show effects in the treatment of dementia and cerebrovascular disorders, EP-A 474580.

Citalopram was first disclosed in DE 2,657,013 corresponding to US 4,136,193. This patent publication describes the preparation of citalopram by one method and outlines a further method which may be used for preparing citalopram.

According to the process described, the corresponding 1-(4-fluorophenyl)-1,3-dihydro-5-isobenzofurancarbonitrile is reacted with 3-(N,N-dimethylamino)propyl-chloride in the presence of methylsulfinylmethide as condensing agent. The starting material was prepared from the corresponding 5-bromo derivative by reaction with cuprous cyanide.

According to the method, which is only outlined in general terms, citalopram may be obtained by ring closure of the compound: 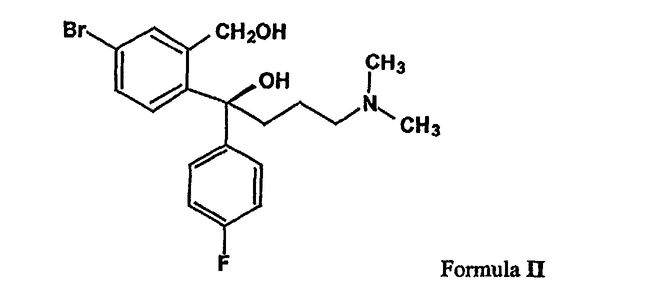 in the presence of a dehydrating agent and subsequent exchange of the 5-bromo group with cyano using cuprous cyanide. The starting material of Formula **II** is obtained from 5-bromophthalide by two successive Grignard reactions, i.e. with 4-fluorophenyl magnesium chloride and N,N-dimethylaminopropyl magnesium chloride, respectively.

A new and surprising method and an intermediate for the preparation of citalopram were described in US Patent No 4,650,884 according to which an intermediate of the formula 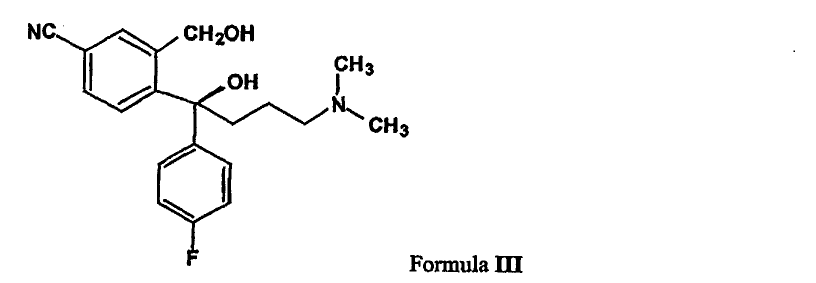 is subjected to a ring closure reaction by dehydration with strong sulfuric acid in order to obtain citalopram. The intermediate of Formula **III** was prepared from 5-cyanophthalide by two successive Grignard reactions, *i*.*e*. with 4-fluorophenyl magnesium halogenide and N,N-dimethylaminopropyl magnesium halogenide, respectively.

Further processes are disclosed in International patent application Nos. WO 98019511, WO 98019512 and WO 98019513. WO 98019512 and WO 98019513 relate to methods wherein a 5-amino-, 5-carboxy- or 5-(sec. aminocarbonyl)phthalide is subjected to two successive Grignard reactions, ring closure and conversion of the resulting 1,3-dihydroisobenzofuran derivative to the corresponding 5-cyano compound, i.e. citalopram. International patent application No. WO 98019511 discloses a process for the manufacture of citalopram wherein a (4-substituted-2-hydroxymethylphenyl-(4-fluorphenyl)methanol compound is subjected to ring closure and the resulting 5-substituted 1-(4-fluorophenyl)-1,3-dihydroisobenzofuran converted to the corresponding 5-cyano derivative which is alkylated with a (3-dimethylamino)propylhalogenide in order to obtain citalopram.

Finally, methods of preparing the individual enantiomers of citalopram are disclosed in US Patent No 4,943,590 from which it also appears that the ring closure of the intermediate of Formula **III** may be carried out via a labile ester with a base.

With respect to the above methods for the preparation of citalopram the proces comprising exchange of the 5-bromo group with cyano proved not to be very convenient in commercial scale, since it was the yield was rather low, the product was impure and in particular that it was difficult to separate the resulting citalopram from the corresponding 5-bromo compound.

It has now been found that citalopram may be obtained in a high yield as a very pure product by a new catalytic process in which 5-cyano is exchanged for a 5-halogen or a 5-triflate group of 1-[3-(dimethylamino)propyl]-1-(4-fluorophenyl)-1,3-dihydro-5-isobenzofuran thus avoiding the extensive work up of the old cyanide exchange process.

### Summary of the invention

Accordingly, the present invention relates to a novel method for the preparation of citalopram comprising reaction of a compound of Formula **IV** 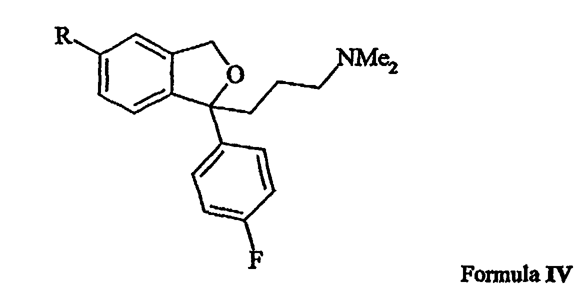 wherein R is iodo, bromo, chloro, or CF₃-(CF₂)ₙ-SO₂-O- wherein n is an integer in the range 0-8, incl., with a cyanide source, for example KCN, NaCN or (R')₄NCN where (R')₄ indicates four groups which may be the same or different and are selected from hydrogen and straight chain or branched C₁₋ ₆ alkyl, in the presence of a palladium catalyst and a catalytic amount of Cu⁺ or Zn²⁺, or with Zn(CN)₂ in the presence a palladium catalyst, and isolation of the corresponding 5-cyano compound, i.e. citalopram 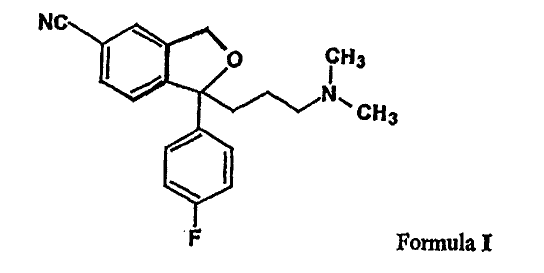 as the base or a pharmaceutically acceptable salt thereof.

In another aspect, the present invention provides the novel intermediates of Formula **IV** wherein R is CF₃-(CF₂)ₙ-SO₂-O- wherein n is an integer in the range 0-8 or R is iodo.

In a further aspect the invention relates to the above process in which the compound of Formula **IV** is the S-enatiomer.

By the process of the invention citalopram is obtained as a pure product in high yield thus reducing costly purification processes. Furthermore, the reaction may be carried out in more convenient solvents, at a low temperature and at a low excess of CN⁻ compared to the known cyano exchange process. The process has environmental advantages in that it only uses small amounts of heavy metal. Finally, this process gives an improved crystalline product enabling easy conversion to desired salts. The intermediates of Formula **IV** wherein R is CF₃-(CF₂)ₙ-SO₂-O- wherein n is an integer in the range 0-8 or R is iodo have been found to show pharmacological activity, i.e. 5-HT reuptake inhibiting effects, and accordingly they are useful as antidepressants

The cyanide source used may be any useful source. Preferred sources are KCN, NaCN or (R')₄NCN where (R')₄ is as defined above. The cyanide source is used in a stoichiometric amount or in excess, preferably 1-2 equivalents are used pr. equivalent starting material of Formula IV. (R')₄N⁺ may conveniently be (Bu)₄N⁺. The cyanide compound is preferably NaCN or KCN or Zn(CN)₂.

The palladium catalyst may be any suitable Pd(0) or Pd(II) containing catalyst, such as Pd(PPh₃)₄, Pd₂(dba)₃, Pd(PPh)₂Cl₂, etc. The Pd catalyst is conveniently used in an amount of 1-10, preferably 2-6, most preferably about 4-5 mol%.

Catalytic amounts of Cu⁺ and Zn²⁺, respectively, means substoichiometric amounts such as 0.1 - 5, preferably 1 - 3 %. Conveniently, about ½ eq. is used per eq. Pd. Any convenient source of Cu⁺ and Zn⁺⁺ may be used. Cu⁺ is preferably used in the form of CuI and Zn²⁺ is conveniently used as the Zn(CN)₂ salt.

In a preferred embodiment of the invention, R is CF₃-(CF₂)ₙ-SO₂-O- wherein n is an integer from the range 0 to 8 or R is bromo or iodo, most preferably CF₃-(CF₂)₈-SO₂-O-, CF₃-SO₂O-, bromo or iodo, in particular bromo.

In another particularly preferred embodiment the compound of Formula IV is reacted with ZnCN₂ in the presence of a Palladium catalyst, preferably Pd(PPh₃)₄ (tetrakis(triphenylphosphine)palladium).

The intermediate of Formula **IV** wherein R is bromo or chloro may be prepared from bromo-and chlorophthalide, respectively, as described in DE 2,657,013 and the corresponding US 4,136,193. The iodo may be prepared analogously from the corresponding phthalide derivatives and the compounds wherein R is CF₃-(CF₂)ₙ-SO₂-O- may be prepared from the corresponding hydroxy compounds by a conventional triflation reaction.

The reaction may be performed in any convenient solvent, preferably acetonitril, propionitrile, THF and ethylacetate.

Other reaction conditions, solvents, etc. are conventional conditions for such reactions and may easily be determined by a person skilled in the art.

The compound of general Formula **I** may be used as the free base or as a pharmaceutically acceptable acid addition salt thereof. As acid addition salts, such salts formed with organic or inorganic acids may be used. Exemplary of such organic salts are those with maleic, fumaric, benzoic, ascorbic, succinic, oxalic, bismethylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzene sulfonic and theophylline acetic acids, as well as the 8-halotheophyllines, for example 8-bromotheophylline. Exemplary of such inorganic salts are those with hydrochloric, hydrobromic, sulfuric, sulfamic, phosphoric and nitric acids.

The acid addition salts of the compounds may be prepared by methods known in the art. The base is reacted with either the calculated amount of acid in a water miscible solvent, such as acetone or ethanol, with subsequent isolation of the salt by concentration and cooling, or with an excess of the acid in a water immiscible solvent, such as ethylether, ethylacetate or dichloromethane, with the salt separating spontaneously.

The pharmaceutical compositions of the invention may be administered in any suitable way and in any suitable form, for example orally in the form of tablets, capsules, powders or syrups, or parenterally in the form of usual sterile solutions for injection.

The pharmaceutical formulations of the invention may be prepared by conventional methods in the art. For example, tablets may be prepared by mixing the active ingredient with ordinary adjuvants and/or diluents and subsequently compressing the mixture in a conventional tabletting machine. Examples of adjuvants or diluents comprise: Corn starch, potato starch, talcum, magnesium stearate, gelatine, lactose, gums, and the like. Any other adjuvant or additive, colourings, aroma, preservatives etc. may be used provided that they are compatible with the active ingredients.

Solutions for injections may be prepared by dissolving the active ingredient and possible additives in a part of the solvent for injection, preferably sterile water, adjusting the solution to the desired volume, sterilisation of the solution and filling in suitable ampoules or vials. Any suitable additive conventionally used in the art may be added, such as tonicity agents, preservatives, antioxidants, etc.

### Examples

The invention is further illustrated by the following examples.

### Example 1

### Citalopram oxalate

### Method 1

A mixture of Zn(CN)₂ (1.2g, 0.01mol) and 1-(4'-fluorophenyl)-1-(3-dimethylaminopropyl)-5-bromophtalane (6.0g, 0.016mol) in DMF (40mL) was stirred at room temperature under an atmosphere of argon for 30 minutes. Dissolved oxygen was removed by bubbling argon through the reaction mixture for 10 minutes and then tetrakis(triphenylphosphine)palladium (0) (0.8g, 0.0007mol, 4.3mol%) was added. Then the reaction mixture was heated at 75 °C for 3 hrs, poured into water (200mL) and extracted with diethyl ether (2 x 100mL), dried (MgSO₄), filtered and concentrated under reduced pressure. The residue was dissolved in acetone (10mL) and a solution of oxalic acid (0.145g, 0.016mol) in acetone (10 mL) was added with stirring. The citalopram oxalate was isolated by filtration, washed with cold diethyl ether and dried in vacuo to pure citalopram, oxalate (6.1 g, 92 %)

### Method 2

A mixture of 1-(4'-fluorophenyl)-1-(3-dimethylaminopropyl)-5-bromophtalane (2.5g, 0.007mol), NaCN (0.68 g, 0.014mol), and Zn(CN)₂ (0.014g, 0.00012mol) in THF (40 mL) were stirred at room temperature under an atmosphere of argon for 30 minutes. Then dissolved oxygen was removed by bubbling argon through the reaction mixture before the addition of tetrakis(triphenylphosphine)palladium (0) (0.3 g, 0.0003 mol, 3.7 mol%). Then the reaction mixture was heated at reflux overnight, cooled, diluted with diethyl ether, and then filtered through celite. The filtrate was washed with brine, dried (MgSO₄) and concentrated under reduced pressure. The residue was dissolved in acetone (50mL) and a solution of oxalic acid (0.63g, 0.007mol) in acetone (10 mL) was added with stirring. The Citalopram oxalate was isolated by filtration, washed with cold diethyl ether and dried in vacuo to pure citalopram, oxalate (2.4g ,82%)

### Example 2

### 1-(4'-fluorphenyl)-1-(3-dimethylaminopropyl)-5-iodophtalane, oxalate.

A solution of4-fluorophenylmagnesium bromide, prepared from 4-fluorobromobenzene (19.3 g, 0.11 mole) and magnesium turnings (2.92 g, 0.12 mol) in dry THF (100 mL), is added dropwise to a suspension of 5-iodophtalide (26.0 g, 0.1 mole) in dry THF (100 mL). The temperature is kept below 0 °C. After the addition is complete, the reaction mixture is stirred for 3 hours at 0 °C. A second Grignard solution prepared from 3-dimethylaminopropyl chloride (14.6 g, 0.12 mole) and magnesium turnings (3.2 g, 0.13 mole) in dry THF (100 mL) is added to the reaction mixture. The temperature is kept below 0 °C during the addition. After the addition is complete the cooling is removed and the reaction mixture is stirred for an additional 2 hours at ambient temperature. The reaction mixture is then poured into a mixture of ice water (200 mL) and a saturated solution of NH₄Cl (100 mL). THF is evaporated in vacuo. Toluene (200 mL) is added and the organic phase is separated and extracted with 1 M HCl (1 x 100 mL). The pH of the water phase is then adjusted to 9 by addition of 25 % NH₄OH (15 mL) and toluene (100 mL) is added. The reaction is left overnight at room temperature.
The organic phase is separated and 70 % sulfuric acid (10 mL) is added at room temperature. The reaction mixture is stirred at room temperature for 2 hours to complete the ring closure. 25 % NH₄OH (20 mL) is added and the organic phase is separated, filtered and evaporated in vacuo to give the crude title compound as its free base.
A sample of the crude material (5.0 g, 11.3 mmol) is dissolved in ethyl acetate and filtered through silica. Eluent 1: Ethyl acetate which is discarded. Eluent 2: Ethyl acetate:Triethyl amine, 95:5 which is collected and evaporated in vacuo to give the title compound (3.5 g, 8.2 mmol) as its free base.
The oxalate salt is precipitated from acetone.
DSC onset: 82 °C and 195 °C. ¹H NMR (DMSO d-6, 250 MHz): 1.3 - 1.65 (2H,m), 2.15 (2H,t, J=10 Hz), 2.63 (6H,s), 2.87 (2H,t, J=10 Hz), 5.0-5.2 (2H, 2d, J= 12.5 Hz), 6.5 - 7.05 (2H,s (broad)), 7.16 (2H,t, J=7.5 Hz), 7.35 (1H,d, J=8.5 Hz), 7,55 (2H,dt, J= 1.2 Hz, J=7.5 Hz), 7.64 (1H,d, J=8.5 Hz), 7.69 (1H,s).

### Example 3

### 1-(3-Dimethylamino-1-propyl)-1-(4-fluorophenyl)-5-hydroxy-1,3-dihydroisobenzofurane, oxalate

A solution of 4-fluorophenylmagnesium bromide, prepared from 4-fluorobromobenzene (24,0 g, 0,14 mole) and magnesium turnings (4,38 g, 0,17 mole) in dry THE (80 mL), is added dropwise to a suspension of 5-hydroxyphthalide (10,0 g, 0,07 mole) in dry THF (100 mL) at a temperature below 8°C. The reaction mixture is stirred at room temperature overnight after the addition is finished.
A second Grignard solution prepared from 3-dimethylaminopropyl chloride (8,50 g, 0,07 mole) and magnesium turnings (1,93 g, 0,07 mole) in dry THF (40 mL) and added to the reaction mixture while the temperature is keept below 10°C. The reaction is left stirred overnight.
The reaction mixture is poured into ice water (200 mL) and pH is adjusted to 7 with ammonium chloride water (300 mL) resulting in separation of two phases. The water phase is extracted with ethylacetate (300 mL) and then made basic to pH 8 - 9 with 25%(w/v) ammonium hydroxide. The water phase is extracted with toluene/ethylacetate (3:2, 3x100 mL). The toluene extract is dried over anhydrous sodium sulphate and stirred with charcoal. After filtration the solvent is evaporated in vacuo and the title compound is obtained as a oil (10,2 g, 48%).
5,1 grams (16 mmol) of the obtained oil is dissolved in acetone (25 mL) and treated with anhydrous oxalic acid (1,46 g, 0,016 mole). The mixture is left in the freezer overnight and the precipitated oxalate is filtered off. Yield: 4,77 g
DSC onset 168°C. ¹H NMR (DMSO-d₆, 500 MHz): 1,36 - 1,58 (2H, m), 2,05 - 2,18 (2H, m), 2,63 (6H, s), 2,96 (2H, t, J = 6,5 Hz), 4,95 (1H, d, J = 12,5 Hz), 5,08 (1H, d, J = 12,5 Hz), 6,65 (1H, s), 6,70 (1H, d, J = 8,5 Hz), 7,14 (2H, t, J = 7,5 Hz), 7,24 (1H, d, J = 8,5 Hz) 7,52 (2H, dt, J = 7,5 J = 1,2 Hz), 9 - 10 (2H, broad s).
Anal. calc. for C₂₁H₂₄N₁F₁O₆: C, 62,20; H, 5,98: N, 3,46.
Found: C, 62,02; H, 5,97; N, 3,42.

### Example 4

### 1-(3-Dimethylamino-1-propyl)-1-(4-fluorophenyl)-5-[(trifluoromethyl)sulfonyl-oxy]-1,3-dihydroisobenzofurane, oxalate.

1-(3-Dimethylamino-1-propyl)-1-(4-fluorophenyl)-5-hydroxy-1,3-dihydroisobenzofurane (1,79 g, 5,7 mmol) is dissolved in dichloromethane (35 ml) and cooled in ice/water bath. Under nitrogen trifluoromethane sulfonic acid chloride (0,73 ml, 6,8 mmol) is added dropwise keeping the temperature below 5°C. The reaction mixture is allowed to warm to room temperature overnight.
Water (40 mL) and triethylamine (1 mL) are added and the phases are separated. The water phase is extracted with dichloromethane (25 mL). The combined organic phases are dried over magnesium sulphate and the solvent evaporated in vacuo. The residue (2,09 grams of the title compound as its free base) is dissolved in acetone (10 mL) and treated with anhydrous oxalic acid (0,51 g, 5,7 mmol). After stirring at room temperature overnight the precipitate is filtered off. Yield: 0,84 g, 33%.
DSC onset 144°C. ¹H NMR (DMSO-d₆, 500 MHz): 1,37 - 1,57 (2H, m), 2,15 - 2,25 (2H, m), 2,61 (6H, s), 2,95 (2H, t, J = 9,4 Hz), 5,12 (1H, d, J = 12,5 Hz), 5,22 (1H, d, J = 12,5 Hz), 7,17 (2H, t, J = 6,3 Hz), 7,42 (1H, d, J = 7,8 Hz), 7,48 (1H, s), 7,59 (2H, dt, J = 6,3 Hz J = 1,2 Hz), 7,70 (1H, d, J = 7,8 Hz).
Anal. calc. for C₂₂H₂₃N₁F₄O₈S₁: C, 49,16; H, 4,32: N, 2,61.
Found: C, 49,43; H, 4,36; N, 2,57.

### Example 5

### Citalopram, oxalate, Method 3

1-(3-Dimethylamino-1-propyl)-1-(4-fluorophenyl)-5-[(trifluoromethyl)sulfonyl-oxy]-1,3-dihydroisobenzofurane (1,02 g, 2,3 mmol), sodium cyanide (0,22 g, 4,6 mmol), copper iodide (0,05 g, 0,3 mmol) and tetrakis(triphenylphosphine)palladium(0) (0,125 g, 0,1 mmol) are suspended in acetonitrile (10 mL). The suspension is heated at reflux for 5 hours and then allowed to cool to room temperature overnight with intensive stirring. Ethylacetate (30 mL) is added and the mixture is filtrated on celite. The filtrate is washed with brine (60 mL) and dried over magnesium sulphate before the solvent is removed in vacuo. The crude product is eluted on silica (eluent: ethylacetate, ethanol, triethylamine 75:25:4). Yield: 0,22 g, 30%.
The oxalate salt is precipitated from acetone.

## Claims

1. A method for the preparation of citalopram comprising reaction of a compound of Formula **IV** 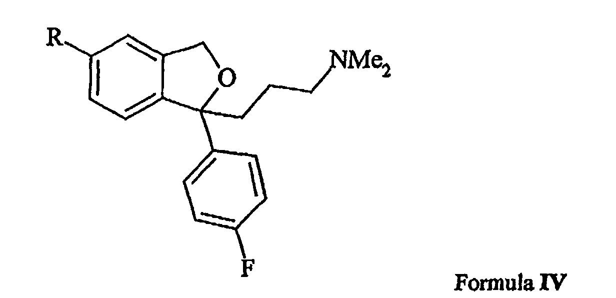 wherein R is halogen, or CF₃-(CF₂)ₙ-SO₂-O- wherein n is an integer in the range 0-8, incl., with a cyanide source in the presence of a palladium catalyst and a catalytic amount of Cu⁺ or Zn²⁺, or with Zn(CN)₂ in the presence a palladium catalyst, and isolation of the corresponding 5-cyano compound, i.e. citalopram 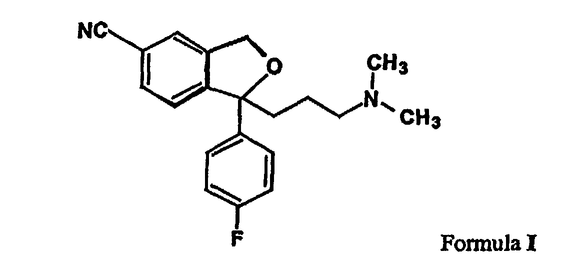 as the base or a pharmaceutically acceptable salt thereof.

2. The method of Claim 1, wherein the cyanide source is KCN, NaCN or (R')₄NCN where (R')₄ indicates four groups which may be the same of different and are selected from hydrogen and straight chain or branched C₁₋₆ alkyl,

3. The method of Claim 1 or 2 wherein R is CF₃-(CF₂)ₙ-SO₂-O- wherein n is an integer from the range 0 to 8, preferably CF₃-SO₂-O-.

4. The method of Claim 1 or 2 wherein R is bromo or iodo.

5. The method of any of Claims 1, 3 - 4 wherein the compound of Formula IV is reacted with ZnCN₂ in the presence of a palladium catalyst, preferably Pd(PPh₃)₄.

6. The method of any of Claims 1, 3 - 4 wherein the cyanide compound used is NaCN, KCN or Zn(CN)₂.

7. The method of any of Claims 1 - 4 and 6 wherein the palladium catalyst is Pd(PPh₃)₄, Pd₂(dba)₃ or Pd(PPh)₂Cl₂.

8. The method of Claim 7 wherein the palladium catalyst is Pd(PPh₃)₄.

9. The method of any of Claims 1 - 8 wherein the reaction is carried out in the presence of a catalytic amount of Cu⁺, preferably in the form of CuI.

10. The method of any of Claims 1 - 8 wherein the reaction is carried out in the presence of a catalytic amount of Zn²⁺, preferably as Zn(CN)₂.

11. A compound of Formula **IV** 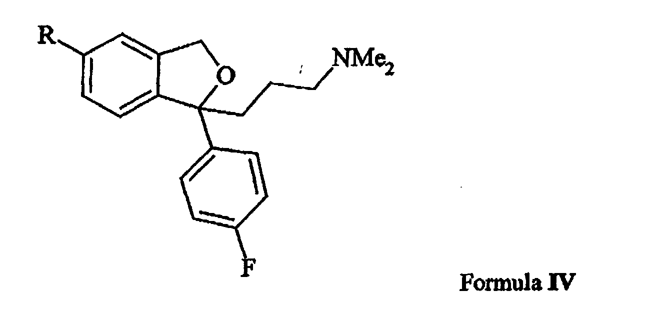 wherein R is CF₃-(CF₂)ₙ-SO₂-O- wherein n is an integer in the range 0-8 or R is iodo.

12. The method of any of Claims 1 - 10 wherein the compound of Formula **IV** is the S-enantiomer.

13. The use of a compound according to claim 11 for the preparation of citalopram base or a pharmaceutically acceptable salt thereof.

14. The use of a compound according to claim 11 which is the S-enantiomer for the preparation of S-citalopram or a pharmaceutically acceptable salt thereof.

## Patentansprüche

1. Verfahren zur Herstellung von Citalopram, umfassend die Reaktion einer Verbindung der Formel (IV): 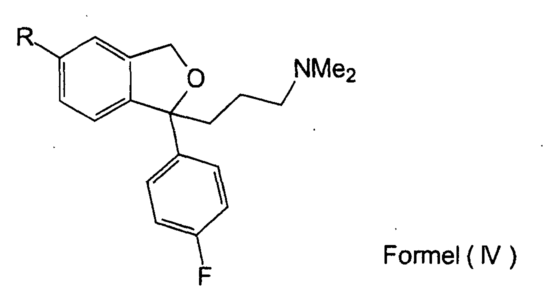 worin R Halogen oder CF₃-(CF₂)ₙ-SO₂-O- ist, worin n eine ganze Zahl im Bereich von 0 bis 8 einschliesslich ist, mit einer Cyanidquelle in Gegenwart eines Palladiumkatalysators und einer katalytischen Menge von Cu⁺ oder Zn²⁺ oder mit Zn(CN)₂ in Gegenwart eines Palladiumkatalysators und Isolierung der entsprechenden 5-Cyanoverbindung, d.h. Citalopram 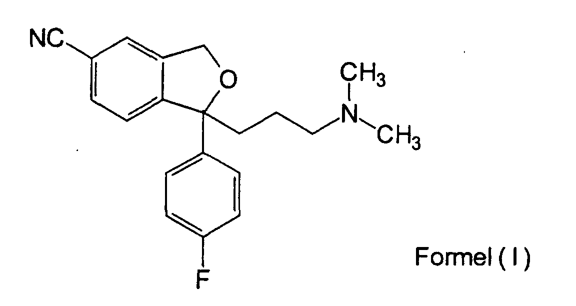 als Base oder pharmazeutisch akzeptables Salz davon.

2. Verfahren gemäss Anspruch 1, worin die Cyanidquelle KCN, NaCN oder (R')₄NCN ist, worin (R')₄ vier Gruppen angibt, die gleich oder verschieden sein können und aus Wasserstoff und geradkettigem oder verzweigtem C₁₋₆-Alkyl ausgewählt sind.

3. Verfahren gemäss Anspruch 1 oder 2, worin R CF₃-(CF₂)ₙ-SO₂-O- ist, worin n eine ganze Zahl aus dem Bereich von 0 bis 8 ist, bevorzugt CF₃-SO₂-O-.

4. Verfahren gemäss Anspruch 1 oder 2, worin R Brom oder Iod ist.

5. Verfahren gemäss einem der Ansprüche 1, 3 bis 4, worin die Verbindung der Formel (IV) mit ZnCN₂ in Gegenwart eines Palladiumkatalysators, bevorzugt Pd(PPh₃)₄, umgesetzt wird.

6. Verfahren gemäss einem der Ansprüche 1, 3 bis 5, worin die verwendete Cyanidverbindung NaCN, KCN oder Zn(CN)₂ ist.

7. Verfahren gemäss einem der Ansprüche 1 bis 4 und 6, worin der Palladiumkatalysator Pd(PPh₃)₄, Pd₂(dba)₃ oder Pd(PPh)₂Cl₂ ist.

8. Verfahren gemäss Anspruch 7, worin der Palladiumkatalysator Pd(PPh₃)₄ ist.

9. Verfahren gemäss einem der Ansprüche 1 bis 8, worin die Reaktion in Gegenwart einer katalytischen Menge von Cu⁺ durchgeführt wird, bevorzugt in Form von CuI.

10. Verfahren gemäss einem der Ansprüche 1 bis 8, worin die Reaktion in Gegenwart einer katalytischen Menge von Zn²⁺ durchgeführt wird, bevorzugt als Zn(CN₂).

11. Verbindung der Formel (IV): 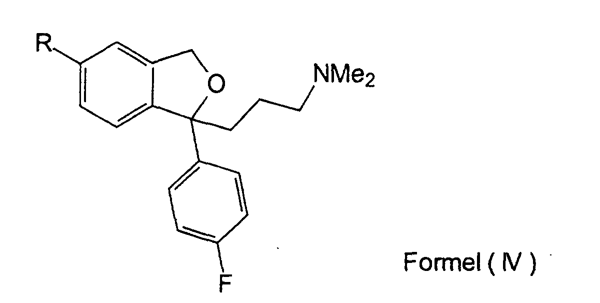 worin R CF₃-(CF₂)ₙ-SO₂-O- ist, worin n eine ganze Zahl im Bereich von 0 bis 8 ist, oder R Iod ist.

12. Verfahren gemäss einem der Ansprüche 1 bis 10, worin die Verbindung der Formel (IV) das S-Enantiomer ist.

13. Verwendung einer Verbindung gemäss Anspruch 11 zur Herstellung von Citalopram-Base oder einem pharmazeutisch akzeptablen Salz davon.

14. Verwendung einer Verbindung gemäss Anspruch 11, die das S-Enantiomer ist, zur Herstellung von S-Citalopram oder einem pharmazeutisch akzeptablen Salz davon.

## Revendications

1. Procédé de préparation de citalopram comprenant la réaction d'un composé de Formule IV 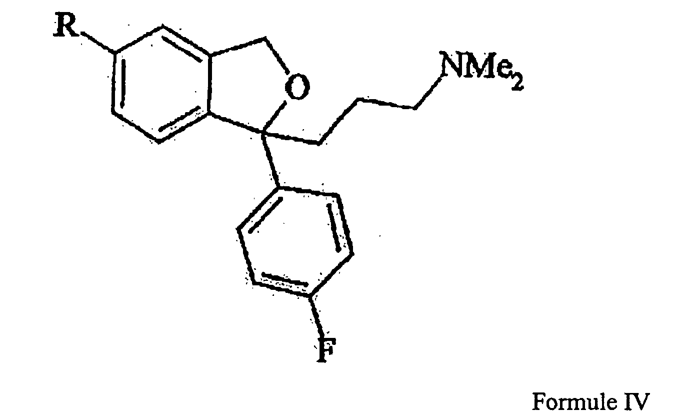 dans lequel R est un halogène, ou CF₃-(CF₂)ₙ-SO₂-O- dans lequel n est un entier dans la fourchette de 0 à 8 inclus, avec une source cyanure en présence d'un catalyseur de palladium et d'une quantité catalytique de Cu⁺ ou de Zn²⁺, ou avec Zn(CN)₂ en présence d'un catalyseur de palladium, et l'isolation du composé 5-cyano correspondant, c'est-à-dire le citalopram 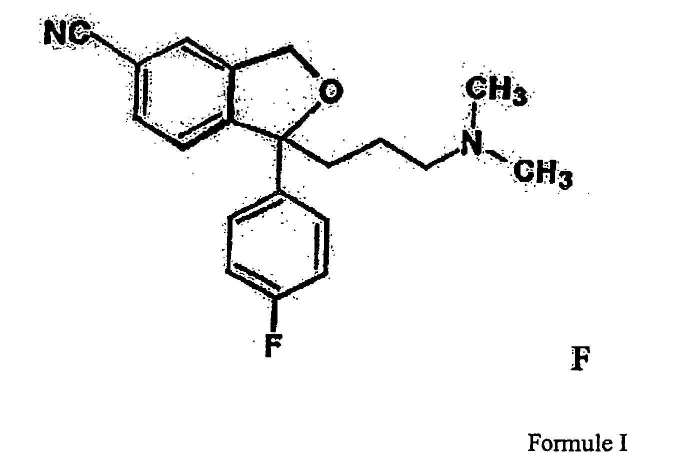 sous la forme d'une base ou d'un sel pharmaceutiquement acceptable de celle-ci.

2. Procédé selon la revendication 1, dans lequel la source cyanure est KCN, NaCN ou (R')₄NCN où (R')₄ désigne quatre groupes qui peuvent être identiques ou différents, et sont sélectionnés à partir de l'hydrogène et d'un alkyl C ₁₋₆ à chaîne droite ou ramifiée.

3. Procédé selon la revendication 1 ou 2, dans lequel R est CF₃-(CF₂)ₙ-SO₂-O-, dans lequel n est un entier dans la fourchette de 0 à 8, de préférence CF₃-SO₂-O-.

4. Procédé selon la revendication 1 ou 2, dans lequel R est bromo ou iodo.

5. Procédé selon l'une quelconque des revendications 1, 3 - 4, dans lequel le composé de Formule IV est soumis à une réaction avec ZnCN₂ en présence d'un catalyseur de palladium, de préférence Pd(PPh₃)₄.

6. Procédé selon l'une quelconque des revendications 1, 3 - 4, dans lequel le composé cyanure utilisé est NaCN, KCN ou Zn(CN)₂.

7. Procédé selon l'une quelconque des revendications 1 - 4 et 6, dans lequel le catalyseur de palladium est Pd(PPh₃)₄, Pd₂(dba)₃ ou Pd(PPh)₂Cl₂.

8. Procédé selon la revendication 7, dans lequel le catalyseur de palladium est Pd(PPh₃)₄.

9. Procédé selon l'une quelconque des revendications 1 - 8, dans lequel la réaction est conduite en présence d'une quantité catalytique de Cu⁺, de préférence sous la forme CuI.

10. Procédé selon l'une quelconque des revendications 1 - 8, dans lequel la réaction est conduite en présence d'une quantité catalytique de Zn²⁺, de préférence comme Zn(CN)₂.

11. Composé de Formule IV 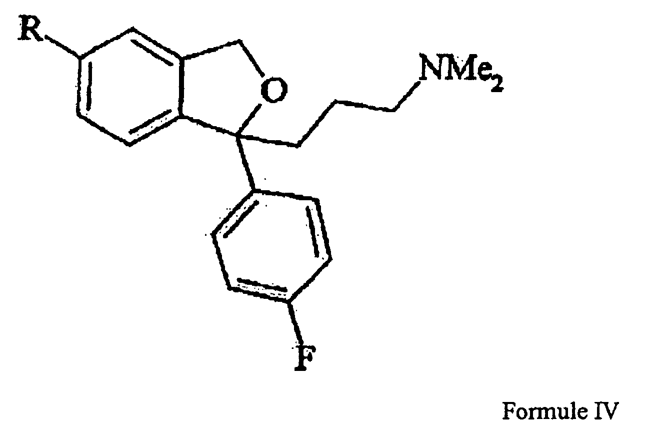 dans lequel R est CF₃-(CF₂)ₙ-SO₂-O-, dans lequel n est un entier dans la fourchette de 0 à 8, ou R est iodo.

12. Procédé selon l'une quelconque des revendications 1 - 10, dans lequel le composé de Formule IV est l'énantiomère-S.

13. Utilisation d'un composé selon la revendication 11, pour la préparation de la base de citalopram, ou d'un sel pharmaceutiquement acceptable de celle-ci.

14. Utilisation d'un composé selon la revendication 11, qui est l'énantiomère-S pour la préparation de S-citalopram, ou d'un sel pharmaceutiquement acceptable de celui-ci.
